# DEMANDE DE BREVET EUROPEEN

(11) **EP 2 295 050 A1**
(43) Date de publication de la demande: **16.03.2011**
(21) Numéro de dépôt: 10011610.2
(22) Date de dépôt: 08.09.2006
(51) Int. Cl.: A61K 31/165, A61P 25/24

(54) **Utilisation de l' agomélatine pour l' obtention de médicaments destinés au traitement des troubles du sommeil chez le patient déprimé**

(30) Priorité: 09.09.2005 FR 0509207
(62) Demande divisionnaire de: 06291422.1
(71) Demandeur: Les Laboratoires Servier, 92284 Suresnes Cedex (FR)
(72) Inventeur: Delalleau, Bruno, 92600 Asnieres-sur-Seine (FR)

(57) **Abrégé**

La présente invention concerne l'utilisation de l'agomélatine ou *N*-[2-(7-méthoxy-1-naphtyl)éthyl]acétamide pour l'obtention de médicaments destinés au traitement des troubles du sommeil chez le patient déprimé.

## Description

La présente invention concerne l'utilisation de l'agomélatine ou *N*-[2-(7-méthoxy-1-naphtyl)éthyl]acétamide de formule (I) : 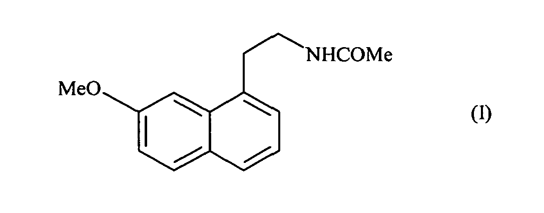 ou un de ses hydrates, formes cristallines et sels d'addition à un acide ou à une base pharmaceutiquement acceptable, seule ou en association pour l'obtention de médicaments destinés au traitement des troubles du sommeil chez le patient déprimé.

L'agomélatine ou *N*-[2-(7-méthoxy-1-naphtyl)éthyl]acétamide présente la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement du trouble dépressif majeur, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285.

La demanderesse a présentement découvert que l'agomélatine ou *N*-[2-(7-méthoxy-1-naphtyl)éthyl]acétamide ou un de ses hydrates, formes cristallines et sels d'addition à un acide ou à une base pharmaceutiquement acceptable, possédait des propriétés intéressantes permettant de l'utiliser dans le traitement des troubles du sommeil chez le patient déprimé.

Le sommeil constitue le rythme biologique circadien le plus marqué chez l'être humain. Ce rythme est gravement perturbé chez le patient déprimé et il n'existe pas actuellement de traitement satisfaisant et reconnu pour palier ces troubles. Les traitements antidépresseurs actuels (fluoxétine, paroxétine, venlafaxine...) ont peu ou pas d'effet spécifique sur les troubles du sommeil chez le patient déprimé. Ceux qui sont actifs le sont par une action sédative puissante et non spécifique conduisant à une diminution des capacités cognitives diurnes des sujets traités. C'est le cas pour la miansérine ou la mirtazapine par exemple (Ridout et al. 2001, Fawcett et al. 1998).

La demanderesse a présentement découvert que l'agomélatine était particulièrement bien adaptée pour le traitement des troubles du sommeil chez le patient déprimé. En effet, l'amélioration du sommeil chez le patient déprimé ne doit pas se faire au détriment de l'architecture du sommeil : les antidépresseurs à action sédative non spécifique perturbent cette architecture, notamment par l'altération du sommeil paradoxal ou du sommeil lent profond (Zarifian et al. 1982). Au contraire, la demanderesse a présentement découvert que l'agomélatine ne se comporte pas comme un antidépresseur classique, et agit tout en respectant l'architecture du sommeil et donc la réaction homéostatique du sommeil chez le patient déprimé.

Ces résultats permettent d'envisager son utilisation, même prolongée, dans les troubles du sommeil chez le patient déprimé.

L'invention concerne donc l'utilisation de l'agomélatine ou un de ses hydrates, formes cristallines et sels d'addition à un acide ou à une base pharmaceutiquement acceptable, pour l'obtention de compositions pharmaceutiques destinées au traitement des troubles du sommeil chez le patient déprimé.

Les compositions pharmaceutiques seront présentées sous des formes convenant aux administrations par voie orale, parentérale, transcutanée, nasale, rectale, perlinguale, et notamment sous forme de préparations injectables, comprimés, comprimés sublinguaux, glossettes, gélules, capsules, tablettes, suppositoires, crèmes, pommades, gels dermiques, etc...

Outre l'agomélatine, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

**A titre d'exemple et de manière non limitative, on peut citer :**
◆ *our les diluants :* le lactose, le dextrose, le sucrose, le mannitol, le sorbitol, la cellulose, la glycérine,
◆ *pour les lubrifiants :* la silice, le talc, l'acide stéarique et ses sels de magnésium et de calcium, le polyéthylène glycol,
◆ *pour les liants :* le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants :* l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne entre 1 mg et 50 mg d'agomélatine par 24 heures.

De manière préférentielle, la dose journalière d'agomélatine sera de 25 mg par jour.

### Composition pharmaceutique :

Formule de préparation pour 1000 comprimés doses à 25 mg :
Agomélatine....................................................................................................................... 25 g
Lactose monohydrate............................................................................................... 62 g
Stéarate de Magnésium............................................................................................ 1,3 g
Povidone .................................................................................................................. 9 g
Silice colloïdale anhydre ......................................................................................... 0,3 g
Cellulose sodium glycolate......:............................................................................... 30 g
Acide stéarique ........................................................................................................ 2,6 g

### Etudes cliniques :

L'action propre de l'agomélatine sur les troubles du sommeil du déprimé est établie en comparaison avec la mirtazapine. Cette étude, réalisée en double-aveugle sur 6 semaines de traitement, a comme critère d'efficacité principal l'enregistrement polysomnographique de sommeil. L'échelle de dépression d'Hamilton permet de documenter l'efficacité antidépressive. Les résultats obtenus montrent que l'agomélatine est particulièrement efficace dans le traitement des troubles du sommeil du patient déprimé.

Une autre étude évaluant l'amélioration du sommeil a été réalisée chez des patients présentant un épisode dépressif caractérisé, en double aveugle sur 6 semaines *versus* venlafaxine. Après une semaine de placebo, 332 patients ont été randomisés : 165 ont reçu 25 mg/jour d'agomélatine et 167 ont reçu 75 mg/jour de venlafaxine pendant 2 semaines. Après 2 semaines de traitement, si la réponse clinique était insuffisante, certains patients ont eu leur traitement doublé à 50 mg/jour d'agomélatine ou 150 mg/jour de venlafaxine les semaines suivantes.

L'échelle de dépression d'Hamilton permet de documenter l'efficacité antidépressive et les résultats obtenus ont montré une efficacité comparable de l'agomélatine versus venlafaxine.

Les effets sur le sommeil ont été évalués avec le questionnaire d'évaluation du sommeil de Leeds (LSEQ, Leeds Sleep Evaluation Questionnaire) : dans l'item « facilité à s'endormir » (GTS, ease of Getting To Sleep), les effets de l'agomélatine ont été plus importants et d'apparition plus rapide que pour la venlafaxine avec une différence significative dès la première semaine (p = 0,007) et restent en faveur de l'agomélatine durant les 6 semaines de traitement. Concernant l'item « qualité du sommeil » (QOS, Quality Of Sleep), l'agomélatine a montré une efficacité significativement plus grande que pour la venlafaxine (p = 0,015). Une amélioration également significative (p inférieur ou égal à 0,001) en faveur de l'agomélatine *versus* venlafaxine a été reportée pour les phénomènes de « somnolences en journée » et la « sensation de bien-être ».

Ces résultats attestent de la supériorité de l'efficacité de l'agomélatine dans le traitement du sommeil chez le patient déprimé.

Enfin, les effets de l'agomélatine sur les paramètres polysomnographiques du sommeil ont été évalués dans une étude pilote réalisée chez 15 patients souffrant d'un épisode dépressif caractérisé. Une restauration de l'architecture physiologique du sommeil a été observée avec l'agomélatine 25 mg. Les durées absolue et relative des stades 3 et 4 de l'enregistrement de l'activité électroencéphalographique sont augmentées de façon significative sans altération du sommeil REM (rapid eye movements). De plus, l'agomélatine augmente la durée total du sommeil, réduit le nombre de réveils nocturnes, et par conséquent améliore l'efficacité du sommeil. Ces effets bénéfiques ont été observés dès la première semaine de traitement.

## Revendications

1. Utilisation de l'agomélatine ou *N*-[2-(7-méthoxy-1-naphtyl)éthyl]acétamide ou un de ses hydrates, formes cristallines et sels d'addition à un acide ou à une base pharmaceutiquement acceptable, pour l'obtention d'un médicament destiné au traitement des troubles du sommeil chez le patient déprimé.

2. Compositions pharmaceutiques contenant de l'agomélatine ou *N*-[2-(7-méthoxy-1-naphtyl)éthyl]acétamide ou un de ses hydrates, formes cristallines et sels d'addition à un acide ou à une base pharmaceutiquement acceptable, seule ou en combinaison avec un ou plusieurs excipients pharmaceutiquement acceptables.

3. Compositions pharmaceutiques selon la revendication 2 utiles pour la fabrication d'un médicament pour le traitement des troubles du sommeil chez le patient déprimé.
